# EUROPEAN PATENT APPLICATION

(11) **EP 4 613 781 A1**
(43) Date of publication of application: **10.09.2025**
(21) Application number: 23896909.1
(22) Date of filing: 30.11.2023
(51) Int. Cl.: C07K 19/00, C12N 15/62, C12N 5/10, C12N 15/86, A61K 35/62, A61K 35/17, A61P 35/00, A61P 31/12

(54) **IMMUNE REJECTION-RESISTANT ENGINEERED CELL**

(30) Priority: 01.12.2022 CN 202211543531
(71) Applicant: Nanjing Bioheng Biotech Co., Ltd, Nanjing, Jiangsu 210061 (CN)
(72) Inventor: ZHOU, Yali, Nanjing, Jiangsu 210061 (CN); CHEN, Gong, Nanjing, Jiangsu 210061 (CN); HUANG, Jie, Nanjing, Jiangsu 210061 (CN); TANG, Yingxiu, Nanjing, Jiangsu 210061 (CN); REN, Jiangtao, Nanjing, Jiangsu 210061 (CN)
(74) Representative: Rimini, Rebecca
(86) International application number: PCT/CN2023/135659
(87) International publication number: WO 2024/114767

(57) **Abstract**

The present invention relates to an immunosuppressive molecule, comprising an immunosuppressive protein binding domain, a transmembrane domain, and a co-stimulatory domain and not comprising a primary signaling domain, wherein the immunosuppressive protein binding domain is selected from: an antibody targeting FasL, an antibody targeting IRP60, a ligand of IRP60 or a functional fragment thereof, an antibody targeting SIRPa, a ligand of SIRPa or a functional fragment thereof, wherein the immunosuppressive molecule can reduce immune rejection by cells of a subject. The present invention further relates to an engineered cell expressing the immunosuppressive molecule and a composition comprising the cell. Further provided in the present invention is a method for reducing immune rejection using the immunosuppressive molecule.

## Description

### CROSS-REFERENCE TO RELATED APPLICATION

The present application claims the priority to the Chinese patent application with the application No. 202211543531.6, and entitled "IMMUNE REJECTION-RESISTANT ENGINEERED CELL", filed with China National Intellectual Patent Administration on December 01, 2022, which is incorporated in the present application by reference in its entirety.

### TECHNICAL FIELD

The present invention pertains to the field of immunotherapy. More specifically, the present invention relates to an immunosuppressive molecule, comprising an immunosuppressive protein binding domain, a transmembrane domain, and a co-stimulatory domain and not comprising a primary signaling domain, wherein the immunosuppressive protein binding domain is selected from: an antibody targeting FasL, an antibody targeting IRP60, a ligand of IRP60 or a functional fragment thereof, an antibody targeting SIRPa, a ligand of SIRPa or a functional fragment thereof, wherein the immunosuppressive molecule can reduce immune rejection by cells of a subject. The present invention further relates to an engineered cell expressing the immunosuppressive molecule, and a method for suppressing immune rejection by cells of a subject.

### BACKGROUND ART

In the case of allogeneic transplantation, exogenous transplanted cells or transplanted organs will be recognized and attacked by immune cells in a subject, resulting in an immune rejection reaction. For example, when a patient receives allogeneic cell therapy (such as CAR-T cells) or organ transplantation, the normal immune system in the patient will reject exogenous cells, which in turn results in host-versus-graft disease (HvGD). At present, HvGD is mainly reduced or avoided by knocking out CD52 or HLA class I molecules. Specifically, knockout of CD52 can make CAR-T cells resistant to alemtuzumab (CD52 antibody), thereby avoiding killing the introduced CAR-T cells when using alemtuzumab to eliminate T cells in the patient. However, the use of alemtuzumab will increase the production cost of a CAR-T product. On the other hand, although knockout of HLA class I molecules can ensure that CAR-T cells are avoided from being eliminated by T-cells in the patient without using an antibody or other treatments, cells with HLA class I molecules knockout will still be recognized by other immune cells of the patient, for example NK cells, and a rejection reaction will occur. Therefore, there is still a need to improve the existing cell therapy, particularly to reduce the immune rejection reaction by a patient.

### SUMMARY

Unless otherwise specified, all scientific and technical terms used herein have the same meaning as commonly understood by one of ordinary skill in the art to which the present invention pertains.

### Immunosuppressive molecules

In a first aspect, the present invention provides an immunosuppressive molecule, comprising an immunosuppressive protein binding domain, a transmembrane domain, and a co-stimulatory domain and not comprising a primary signaling domain, wherein the immunosuppressive protein binding domain is selected from: an antibody targeting FasL, an antibody targeting IRP60, a ligand of IRP60 or a functional fragment thereof, an antibody targeting SIRPa, a ligand of SIRPa or a functional fragment thereof, wherein the immunosuppressive molecule can reduce immune rejection by cells of a subject.

As used herein, the term "immunosuppressive molecule" refers to a molecule capable of binding to an immunosuppressive protein (for example, FasL, IRP60, SIRPα), thereby suppressing immune rejection of exogenous cells by a subject, for example, reducing the killing function of immune cells (e.g., T cells and NK cells) in the subject or suppressing the excessive proliferation of immune cells.

As used herein, the term "antibody" has the broadest meaning understood by those skilled in the art and includes monoclonal antibodies (including whole antibodies), polyclonal antibodies, multivalent antibodies, multispecific antibodies (for example, bispecific antibodies), and antibody fragments or synthetic polypeptides carrying one or more CDR sequences capable of exhibiting the desired biological activity. The antibodies of the present invention may be of any class (for example, IgG, IgE, IgM, IgD, IgA, etc.) or subclass (for example, IgG1, IgG2, IgG2a, IgG3, IgG4, IgA1, IgA2, etc.).

As used herein, the term "antigen-binding fragment" or "antibody fragment" refers to one or more fragments of an antibody that retain the ability to specifically bind an antigen. It has been shown that the antigen-binding function of an antibody can be performed by the fragment of a full-length antibody. Examples of antibody fragments of the present invention include, but are not limited to: Fab, Fab', F(ab')2, Fd fragment, Fd', Fv fragment, single-chain antibody (scFv), disulfide-linked Fv (sdFv), antibody heavy chain variable region (VH) or light chain variable region (VL), linear antibody, "diabody" with two antigen binding sites, single domain antibody (sdAb), nanobody, etc. Therefore, unless the context clearly dictates otherwise, an "antibody" of the present invention encompasses antibody fragments or antigen-binding fragments as defined above. Therefore, in a preferred embodiment, the antibody of the present invention is selected from the group consisting of IgG, Fab, Fab', F(ab')₂, Fd, Fd', Fv, scFv, sdFv, linear antibody, diabody, sdAb or nanobody, preferably scFv, sdAb or nanobody.

Typically, a whole antibody comprises two heavy chains and two light chains disulfide-linked together, each light chain being disulfide-linked to a respective heavy chain, to form a "Y" configuration. Each heavy chain comprises a heavy chain variable region (VH) and a heavy chain constant region, wherein the heavy chain variable region comprises three complementarity determining regions (CDRs): CDR1-H, CDR2-H and CDR3-H, and the heavy chain constant region comprises three constant domains: CH1, CH2 and CH3. Each light chain comprises a light chain variable region (VL) and a light chain constant region, wherein the light chain variable region comprises three CDRs: CDR1-L, CDR2-L and CDR3-L, and the light chain constant region comprises a constant domain CL. In the heavy/light chain variable regions, the CDRs are separated by more conserved framework regions (FRs). The heavy/light chain variable regions recognize and bind antigen, while the constant regions mediate the binding of the antibody to host tissues or factors, including various cells of the immune system (for example, effector cells) and the first component of the classical complement system.

The precise amino acid sequence boundaries for a given CDR or FR can be readily determined using a number of numbering schemes well known in the art, including: Kabat et al. (1991), "Sequences of Proteins of Immunological Interest," 5th Edition, Public Health Service, National Institutes of Health, Bethesda, MD ("Kabat" numbering scheme); Al-Lazikani et al., (1997) JMB 273, 927-948 ("Chothia" numbering scheme); MacCallum et al., J. Mol. Biol 262:732-745 (1996), "Antibody-antigen interactions: Contact analysis and binding sitetopography," J. Mol. Biol. 262, 732-745" ("Contact" numbering scheme); Lefranc MP et al., "IMGT unique numbering for immunoglobulin and T cell receptor variable domains and Ig superfamily V-like domains," Dev Comp Immunol, 2003 Jan; 27(1):55-77 ("IMGT" numbering scheme); Honegger A and Plückthun A, "Yet another numbering scheme for immunoglobulin variable domains: an automatic modeling and analysis tool," JMol Biol, 2001 Jun 8; 309(3):657-70 ("Aho" numbering scheme); and Martin et al., "Modeling antibody hypervariable loops: a combined algorithm," PNAS, 1989, 86(23):9268-9272 ("AbM" numbering scheme).

The boundaries of a given CDR or FR may vary depending on the scheme used for definition. For example, the Kabat scheme is based on structural alignment, while the Chothia scheme is based on structural information. Both the Kabat and Chothia numbering schemes are based on the sequence length of the most common antibody regions where insertions are provided by caret letters (for example, "30a") and deletions occur in some antibodies. These two schemes place certain insertions and deletions (indels) at different positions, resulting in different numbering. The Contact scheme is based on the analysis of complex crystal structures and is similar in many respects to the Chothia numbering scheme. The AbM scheme is a compromise between the Kabat and Chothia definitions and is based on the scheme used by the AbM antibody modeling software of Oxford Molecular.

Therefore, unless otherwise specified, a "CDR" of a given antibody or region thereof (e.g., variable region thereof) is understood to encompass the CDRs as defined by any of the above schemes or other known schemes. For example, where it is specified that a particular CDR (for example, CDR3) comprises a given amino acid sequence, it is understood that such a CDR may also have the sequence of the corresponding CDR (for example, CDR3) as defined by any of the above schemes or other known schemes. Likewise, unless otherwise specified, FRs for a given antibody or region thereof (e.g., variable region thereof) are understood to encompass FRs as defined by any of the above schemes or other known schemes. Unless otherwise specified, the numbering scheme used herein to define the boundaries of CDRs and FRs adopts the Chothia scheme.

In an embodiment, the antibody of the present invention is a murine antibody, a chimeric antibody, a camelid antibody, a humanized antibody or a human antibody.

In an embodiment, the immunosuppressive protein binding domain comprised in the immunosuppressive molecule of the present invention is an antibody targeting FasL. FasL is a type II cell membrane surface glycoprotein, pertaining to a member of the tumor necrosis factor receptor superfamily (TNFRSF). FasL is distributed on the surface of activated T lymphocytes, NK cells, mononuclear macrophages, etc., and its ligand is Fas. It has been found that the Fas/FasL system is one of the important pathways of cell apoptosis. Specifically, the binding of FasL to Fas will form a death-inducing complex, which in turn activates the caspase signaling pathway, and ultimately leads to cell apoptosis through the phosphorylation of tyrosine and serine in the cells. It is reported that the dysfunction of Fas/FasL is closely related to various clinical diseases, for example, autoimmune diseases (rheumatoid arthritis, systemic lupus erythematosus, etc.), acute immune hyperactivity (caused by organ transplantation, multiple trauma, etc.), chronic immune hyperactivity (Kawasaki's disease, idiopathic pulmonary fibrosis, etc.), chronic infection (EBV infection, inflammation caused by viruses, etc.), lymphoid tumors (T-ALL, T cell lymphomas, etc.), etc.

Anti-FasL antibodies known in the art can be used in the present invention. In an embodiment, the antibody targeting FasL comprises a light chain variable region and a heavy chain variable region, wherein CDR1-L, CDR2-L and CDR3-L comprised in the light chain variable region are the same as CDR1-L, CDR2-L and CDR3-L comprised in SEQ ID NO: 8; wherein CDR1-H, CDR2-H and CDR3-H comprised in the heavy chain variable region are the same as CDR1-H, CDR2-H and CDR3-H comprised in SEQ ID NO: 7 or 13. In an embodiment, CDR1-L comprised in the light chain variable region is as set forth in SEQ ID NO: 4, CDR2-L is as set forth in SEQ ID NO: 5, and CDR3-L is as set forth in SEQ ID NO: 6; CDR1-H comprised in the heavy chain variable region is as set forth in SEQ ID NO: 1, CDR2-H is as set forth in SEQ ID NO: 2, and CDR3-H is as set forth in SEQ ID NO: 3; or CDR1-H is as set forth in SEQ ID NO: 10, CDR2-H is as set forth in SEQ ID NO: 11, and CDR3-H is as set forth in SEQ ID NO: 12.

In an embodiment, the antibody targeting FasL comprises a light chain variable region and a heavy chain variable region, wherein the light chain variable region has at least 90%, at least 91%, at least 92%, at least 93%, at least 94%, at least 95%, at least 96%, at least 97%, at least 98%, at least 99%, or 100% identity to SEQ ID NO: 8, or has modifications of one or more amino acids (for example, at most 1, 2, 3, 4, 5, 6, 7, 8, 9 or 10 amino acids) compared to the amino acid sequence as set forth in SEQ ID NO: 8; wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 7 or 13, or has modifications of one or more amino acids (for example, at most 1, 2, 3, 4, 5, 6, 7, 8, 9 or 10 amino acids) compared to the amino acid sequence as set forth in SEQ ID NO: 7 or 13. Preferably, the modifications are conservative modifications, for example, conservative substitutions, additions and deletions of amino acids. In a preferred embodiment, the antibody of the present invention comprises a heavy chain variable region as set forth in SEQ ID NO: 7 or 13 and a light chain variable region as set forth in SEQ ID NO: 8.

In an embodiment, the antibody targeting FasL has at least 90%, at least 91%, at least 92%, at least 93%, at least 94%, at least 95%, at least 96%, at least 97%, at least 98%, at least 99%, or 100% identity to SEQ ID NO: 9 or 14, or has modifications of one or more amino acids (for example, at most 1, 2, 3, 4, 5, 6, 7, 8, 9 or 10 amino acids) compared to the amino acid sequence as set forth in SEQ ID NO: 9 or 14. Preferably, the antibody targeting FasL is as set forth in SEQ ID NO: 9 or 14.

In an embodiment, the immunosuppressive protein binding domain comprised in the immunosuppressive molecule of the present invention is an antibody targeting SIRPα, a ligand of SIRPα or a functional fragment thereof. SIRPα, also known as signal regulatory protein a or CD172a, which is a transmembrane protein expressed on the surface of macrophages, dendritic cells, NK cells, and nerve cells, and its intracellular region contains an immunoreceptor tyrosine-based inhibitory motif (ITIM). The ligand of SIRPα is CD47, a cell surface protein that transmits a "don't eat me" signal on healthy cells. In addition, CD47 is also widely expressed on various tumor cells as a mechanism to evade immune detection. Many studies have found that inhibition of the CD47-SIRPα signaling pathway in cancer cells (for example, by anti-CD47 antibodies or anti-SIRPα antibodies) can promote phagocytosis of tumor cells by macrophages, ultimately limiting tumor growth.

Anti-SIRPα antibodies known in the art can be used in the present invention. In an embodiment, the antibody targeting SIRPα comprises a light chain variable region and a heavy chain variable region, wherein CDR1-L, CDR2-L and CDR3-L comprised in the light chain variable region are the same as CDR1-L, CDR2-L and CDR3-L comprised in SEQ ID NO: 57; wherein CDR1-H, CDR2-H and CDR3-H comprised in the heavy chain variable region are the same as CDR1-H, CDR2-H and CDR3-H comprised in SEQ ID NO: 56. In an embodiment, CDR1-L comprised in the light chain variable region is as set forth in SEQ ID NO: 53, CDR2-L is as set forth in SEQ ID NO: 54, and CDR3-L is as set forth in SEQ ID NO: 55; CDR1-H comprised in the heavy chain variable region is as set forth in SEQ ID NO: 50, CDR2-H is as set forth in SEQ ID NO: 51, and CDR3-H is as set forth in SEQ ID NO: 52.

In an embodiment, the antibody targeting SIRPα comprises a light chain variable region and a heavy chain variable region, wherein the light chain variable region has at least 90%, at least 91%, at least 92%, at least 93%, at least 94%, at least 95%, at least 96%, at least 97%, at least 98%, at least 99%, or 100% identity to SEQ ID NO: 57, or has modifications of one or more amino acids (for example, at most 1, 2, 3, 4, 5, 6, 7, 8, 9 or 10 amino acids) compared to the amino acid sequence as set forth in SEQ ID NO: 57; wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 56, or has modifications of one or more amino acids (for example, at most 1, 2, 3, 4, 5, 6, 7, 8, 9 or 10 amino acids) compared to the amino acid sequence as set forth in SEQ ID NO: 56. Preferably, the modifications are conservative modifications, for example, conservative substitutions, additions and deletions of amino acids. In a preferred embodiment, the antibody of the present invention comprises a heavy chain variable region as set forth in SEQ ID NO: 56 and a light chain variable region as set forth in SEQ ID NO: 57.

In an embodiment, the antibody targeting SIRPα has at least 90%, at least 91%, at least 92%, at least 93%, at least 94%, at least 95%, at least 96%, at least 97%, at least 98%, at least 99%, or 100% identity to SEQ ID NO: 58, or has modifications of one or more amino acids (for example, at most 1, 2, 3, 4, 5, 6, 7, 8, 9 or 10 amino acids) compared to the amino acid sequence as set forth in SEQ ID NO: 58. Preferably, the antibody targeting SIRPα is as set forth in SEQ ID NO: 58.

In an embodiment, the SIRPα binding domain comprised in the immunosuppressive molecule of the present invention is a ligand of SIRPα, for example, CD47 or a functional fragment thereof (i.e., a fragment having SIRPα-binding function), for example the extracellular region of CD47. In said embodiment, the transmembrane region of the immunosuppressive molecule is preferably the transmembrane region of CD47. Preferably, the immunosuppressive molecule may further comprise a portion of the intracellular region of CD47.

In one embodiment, the ligand of SIRPα has at least 90%, at least 91%, at least 92%, at least 93%, at least 94%, at least 95%, at least 96%, at least 97%, at least 98%, at least 99%, or 100% identity to SEQ ID NO: 69, or has modifications of one or more amino acids (for example, at most 1, 2, 3, 4, 5, 6, 7, 8, 9 or 10 amino acids) compared to the amino acid sequence as set forth in SEQ ID NO: 69. Preferably, the ligand of SIRPα is as set forth in SEQ ID NO: 69.

In an embodiment, the immunosuppressive protein binding domain comprised in the immunosuppressive molecule of the present invention is an antibody targeting IRP60, a ligand of IRP60 or a functional fragment thereof. IRP60, also known as CD300a, is a type I transmembrane inhibitory receptor. IRP60 is widely expressed on the surface of myeloid cells (for example, dendritic cells, mast cells, granulocytes, monocytes) and lymphocytes (T cells, B cells, etc.), and its expression level varies depending on the cell type and the degree of differentiation. IRP60 comprises four ITIMs, and the initiation of its inhibitory signal depends on the phosphorylation of tyrosine residues in the ITIMs. By interacting with a ligand aminophospholipid (for example, PtdEth and/or PtdSer), IRP60 is involved in the regulation of a variety of cell activities, for example, cell growth, proliferation, apoptosis, differentiation, immune response, etc. It is reported that IRP60 is closely related to the development of diseases selected from the group consisting of hematological malignancies (for example, leukemia, lymphoma, etc.), infectious diseases, allergic reactivity, chronic inflammatory response, etc.

Anti-IRP60 antibodies known in the art can be used in the present invention, for example, TX41, TX49, etc. In an embodiment, the antibody targeting IRP60 comprises a light chain variable region and a heavy chain variable region, wherein CDR1-L, CDR2-L and CDR3-L comprised in the light chain variable region are the same as the CDR1-L, CDR2-L and CDR3-L comprised in SEQ ID NO: 66; wherein CDR1-H, CDR2-H and CDR3-H comprised in the heavy chain variable region are the same as CDR1-H, CDR2-H and CDR3-H comprised in SEQ ID NO: 65. In an embodiment, CDR1-L comprised in the light chain variable region is as set forth in SEQ ID NO: 62, CDR2-L is as set forth in SEQ ID NO: 63, and CDR3-L is as set forth in SEQ ID NO: 64; CDR1-H comprised in the heavy chain variable region is as set forth in SEQ ID NO: 59, CDR2-H is as set forth in SEQ ID NO: 60, and CDR3-H is as set forth in SEQ ID NO: 61.

In an embodiment, the antibody targeting IRP60 comprises a light chain variable region and a heavy chain variable region, wherein the light chain variable region has at least 90%, at least 91%, at least 92%, at least 93%, at least 94%, at least 95%, at least 96%, at least 97%, at least 98%, at least 99%, or 100% identity to SEQ ID NO: 66, or has modifications of one or more amino acids (for example, at most 1, 2, 3, 4, 5, 6, 7, 8, 9 or 10 amino acids) compared to the amino acid sequence of SEQ ID NO: 66; wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 65, or has modifications of one or more amino acids (for example, at most 1, 2, 3, 4, 5, 6, 7, 8, 9 or 10 amino acids) compared to the amino acid sequence as set forth in SEQ ID NO: 65. Preferably, the modifications are conservative modifications, for example, conservative substitutions, additions and deletions of amino acids. In a preferred embodiment, the antibody of the present invention comprises a heavy chain variable region as set forth in SEQ ID NO: 65 and a light chain variable region as set forth in SEQ ID NO: 66.

In an embodiment, the antibody targeting IRP60 has at least 90%, at least 91%, at least 92%, at least 93%, at least 94%, at least 95%, at least 96%, at least 97%, at least 98%, at least 99%, or 100% identity to SEQ ID NO: 67, or has modifications of one or more amino acids (for example, at most 1, 2, 3, 4, 5, 6, 7, 8, 9 or 10 amino acids) compared to the amino acid sequence as set forth in SEQ ID NO: 67. Preferably, the antibody targeting IRP60 is as set forth in SEQ ID NO: 67.

As used herein, the term "conservative modification" refers to an amino acid modification that does not significantly affect or alter the binding characteristics of an antibody or antibody fragment comprising the amino acid sequence. These conservative modifications include conservative substitutions, additions and deletions of amino acids. Modifications can be introduced into the chimeric antigen receptor of the present invention by standard techniques known in the art, e.g., site-directed mutagenesis and PCR-mediated mutagenesis. A conservative amino acid substitution is one in which an amino acid residue is replaced by an amino acid residue with a similar side chain. Families of amino acid residues with similar side chains have been defined in the art and include those with basic side chain (for example, lysine, arginine, histidine), acidic side chain (for example, aspartic acid, glutamic acid), uncharged polar side chain (for example, glycine, asparagine, glutamine, serine, threonine, tyrosine, cysteine), non-polar side chain (for example, alanine, valine, leucine, isoleucine, proline, phenylalanine, methionine, tryptophan), beta-branched side chain (for example, threonine, valine, isoleucine) and aromatic side chain (for example, tyrosine, phenylalanine, tryptophan, histidine). Conservative modifications can be selected, for example, on the basis of similarity in polarity, charge, solubility, hydrophobicity, hydrophilicity, and/or the amphipathic nature of the residues involved.

As used herein, the term sequence "identity" means the degree to which two (nucleotide or amino acid) sequences in alignment have the same residue at the same position, and is usually expressed as a percentage. Preferably, identity is determined over the entire length of the sequences being compared. Therefore, two copies of the exact same sequence have 100% identity. Those skilled in the art know that several algorithms can be used to determine sequence identity, for example, Blast (Altschul et al. (1997) Nucleic Acids Res. 25:3389-3402), Blast2 (Altschul et al. (1990) J. Mol. Biol. 215: 403-410), Smith-Waterman (Smith et al. (1981) J. Mol. Biol. 147:195-197) and Clustal W.

As used herein, the term "transmembrane domain" refers to a polypeptide structure that enables the expression of an immunosuppressive molecule on the cell surface, and anchors the immunosuppressive protein binding domain to the cell membrane. The transmembrane domain may be natural or synthetic, and also may be derived from any membrane-bound protein or transmembrane protein. The transmembrane domain is capable of signaling when a target binding domain binds to a target. The transmembrane domain particularly suitable for the present invention can be derived from, for example, a TCR α chain, a TCR β chain, a TCR γ chain, a TCR δ chain, a CD3 ζ subunit, a CD3 ε subunit, a CD3 γ subunit, a CD3 δ subunit, CD28, CD45, CD4, CD5, CD8a, CD9, CD16, CD22, CD33, CD37, CD47, CD64, CD80, CD86, CD94, CD134, CD137, CD154, KIRDS2, OX40, CD2, CD27, CD18, ICOS, 4-1BB, GITR, CD40, BAFFR, HVEM, SLAMF7, NKp80, CD160, BCMA, IL-2R β, IL-2R γ, IL-7R a, ITGA1, VLA1, CD49a, ITGA4, IA4, CD49D, ITGA6, VLA-6, CD49f, ITGAD, CDl ld, ITGAE, CD103, ITGAL, CDlla, LFA-1, ITGAM, CDllb, ITGAX, CDllc, ITGB1, CD29, ITGB2, CD18, LFA-1, ITGB7, TNFR2, DNAM1, SLAMF4, CD84, CD96, CEACAM1, CRT AM, Ly9, CD160, PSGL1, CDIOO, SLAMF6, SLAMF1, SLAMF8, CD162, LTBR, PAG/Cbp, NKp44, NKp30, NKp46, NKG2D or NKG2C. In some embodiments, the transmembrane domain is derived from the following molecules: CD8a, CD4, CD28, 4-1BB, CD47, CD80, CD86, CD152 and PD1. Alternatively, the transmembrane domain may be synthetic and may mainly comprise hydrophobic residues, e.g., leucine and valine. Preferably, the transmembrane domain is derived from CD28, which has at least 70%, preferably at least 80%, more preferably at least 90%, at least 95%, at least 97% or at least 99%, or 100% sequence identity to the amino acid sequence as set forth in SEQ ID NO: 15. Preferably, the transmembrane domain is derived from CD8a, which has at least 70%, preferably at least 80%, more preferably at least 90%, at least 95%, at least 97% or at least 99%, or 100% sequence identity to the amino acid sequence as set forth in SEQ ID NO: 16 or 17.

In an embodiment, the immunosuppressive molecule further comprises a hinge region located between the immunosuppressive protein binding domain and the transmembrane domain. As used herein, the term "hinge region" generally refers to any oligopeptide or polypeptide that functions to link a transmembrane domain to an antibody. Specifically, the hinge region serves to provide greater flexibility and accessibility to the antibody. The hinge region may comprise up to 300 amino acids, preferably 10 to 100 amino acids and most preferably 25 to 50 amino acids. The hinge region may be completely or partially derived from a natural molecule, e.g., completely or partially from the extracellular region of CD8, CD4 or CD28, or completely or partially from an antibody constant region. Alternatively, the hinge region may be a synthetic sequence corresponding to a naturally existing hinge sequence, or may be a completely synthetic hinge sequence. In a preferred embodiment, the hinge region comprises a hinge region moiety of CD8α, CD28, an FcγRIIIα receptor, IgG4 or IgG1, more preferably a hinge of CD8α, CD28 or IgG4. In an embodiment, the hinge region is derived from CD28, which has at least 70%, preferably at least 80%, more preferably at least 90%, 95%, 97% or 99%, or 100% sequence identity to the amino acid sequence as set forth in SEQ ID NO: 27. In an embodiment, the hinge region is derived from CD8a, which has at least 70%, preferably at least 80%, more preferably at least 90%, 95%, 97% or 99%, or 100% sequence identity to the amino acid sequence as set forth in SEQ ID NO: 28 or 29. In an embodiment, the hinge region is derived from IgG4, which has at least 70%, preferably at least 80%, more preferably at least 90%, 95%, 97% or 99%, or 100% sequence identity to the amino acid sequence as set forth in SEQ ID NO: 30.

As used herein, the "co-stimulatory domain" refers to at least one moiety of a protein that mediates intracellular signal transduction to induce an immune reaction, e.g., an effector function, which is an intracellular functional signaling domain from a co-stimulatory molecule, comprising the entire intracellular region of the co-stimulatory molecule, or a functional fragment thereof. A "co-stimulatory molecule" refers to a cognate binding partner that specifically binds to a co-stimulatory ligand, thereby mediating a co-stimulatory response (for example, proliferation and survival). The co-stimulatory signaling domain of any co-stimulatory molecule is suitable for the immunosuppressive molecule as described herein. The co-stimulatory molecule includes, but is not limited to, MHC class 1 molecules, BTLA, and Toll ligand receptors. Non-limiting examples of the co-stimulatory domain of the present invention include, but are not limited to, an intracellular region derived from the following protein: LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, 4-1BB, CD270, CD272, B7-H3, ICOS, CD357, DAP10, DAP12, LAT, NKG2C, SLP76, PD-1, LIGHT, TRIM, ZAP70 and combinations thereof. Preferably, the co-stimulatory domain of the CAR of the present invention is 4-1BB, CD28 or 4-1BB+CD28. In an embodiment, the co-stimulatory domain is derived from 4-1BB, which has at least 70%, preferably at least 80%, more preferably at least 90%, 95%, 97% or 99%, or 100% sequence identity to the amino acid sequence as set forth in SEQ ID NO: 19 or 20. In an embodiment, the co-stimulatory domain is derived from CD28, which has at least 70%, preferably at least 80%, more preferably at least 90%, 95%, 97% or 99%, or 100% sequence identity to the amino acid sequence as set forth in SEQ ID NO: 18.

The immunosuppressive molecule of the present invention does not comprise a primary signaling domain. As used herein, the term "primary signaling domain" refers to a protein structure that acts together to initiate primary signaling upon antigen-receptor binding, which is generally an intracellular sequence of a T cell receptor and a co-receptor. The primary signaling domain generally comprises one or more immunoreceptor tyrosine-based activation motifs (ITAMs). Non-limiting examples of the primary signaling domain of the present invention include, but are not limited to, those derived from FcRγ, FcRβ, CD3γ, CD3δ, CD3ε, CD3ζ, CD5, CD22, CD79a, CD79b, NFAM1, STAM1, STAM2, and CD66d. In an embodiment, the immunosuppressive molecule of the present invention does not comprise a CD3ζ intracellular region. For example, the CD3ζ intracellular region has at least 70%, preferably at least 80%, more preferably at least 90%, 95%, 97% or 99%, or 100% sequence identity to the amino acid sequence as set forth in SEQ ID NO: 21, 22 or 23.

In an embodiment, the immunosuppressive molecule of the present invention further comprises a signal peptide such that when it is expressed in a cell, e.g. a T cell, the nascent protein is directed to the endoplasmic reticulum and subsequently to the cell surface. The core of the signal peptide may contain a long hydrophobic amino acid segment, which has a tendency to form a single α-helix. At the end of the signal peptide, there is usually an amino acid segment capable of being recognized and cleaved by signal peptidase. The signal peptidase may cleave during or after translocation, so as to generate free signal peptide and mature protein. Then, the free signal peptide is digested by a specific protease. Signal peptides that may be used in the present invention are well known to those skilled in the art, for example, signal peptides derived from B2M, CD8α, IgG1, GM-CSFRα, etc. In an embodiment, the signal peptide that can be used in the present invention is derived from B2M, which has at least 70%, preferably at least 80%, more preferably at least 90%, 95%, 97% or 99%, or 100% sequence identity to the amino acid sequence as set forth in SEQ ID NO: 24. In an embodiment, the signal peptide that can be used in the present invention is derived from CD8a, which has at least 70%, preferably at least 80%, more preferably at least 90%, 95%, 97% or 99%, or 100% sequence identity to the amino acid sequence as set forth in SEQ ID NO: 25 or 26.

### Nucleic acids and vectors

The present invention further provides a nucleic acid molecule that comprises a nucleic acid sequence encoding the immunosuppressive molecule of the present invention. Optionally, the nucleic acid molecule may further comprise a nucleic acid sequence encoding a functional exogenous receptor (for example, chimeric antigen receptor, chimeric T cell receptor, etc.).

As used herein, the term "nucleic acid molecule" includes sequences of ribonucleotides and deoxyribonucleotides, e.g., modified or unmodified RNA or DNA, each in single- and/or double-stranded form, linear or circular, or mixtures thereof (including hybrid molecules). Therefore, the nucleic acid according to the present invention includes DNA (e.g., dsDNA, ssDNA, cDNA), RNA (e.g., dsRNA, ssRNA, mRNA, ivtRNA), and combinations or derivatives thereof (e.g. PNA). Preferably, the nucleic acid is DNA or RNA.

A nucleic acid may comprise conventional phosphodiester bonds or unconventional bonds (e.g., amide bonds, e.g., those found in peptide nucleic acids (PNAs)). The nucleic acid of the present invention may further comprise one or more modified bases, e.g., for example tritylated bases and unusual bases (e.g. inosine). Other modifications are also contemplated, including chemical, enzymatic or metabolic modifications, so long as the multi-chain CAR of the present invention can be expressed from the polynucleotide. The nucleic acid may be provided in isolated form. In an embodiment, the nucleic acid may further comprise regulatory sequences, e.g., transcriptional control elements (including promoters, enhancers, operators, repressors, and transcriptional termination signals), ribosome binding sites, introns, etc.

The nucleic acid sequences of the present invention may be codon-optimized for optimal expression in desired cells (e.g., immune cells); or for expression in bacteria, yeast or insect cells. Codon optimization refers to the replacement of codons in the target sequence that are generally rare in highly expressed genes of a given species with codons that are generally common in highly expressed genes of this species, while the codons before and after the replacement code for the same amino acid. Therefore, the choice of optimal codons depends on the codon usage bias of the host genome.

The present invention further provides a vector comprising the nucleic acid molecule of the present invention. Optionally, the nucleic acid sequence encoding the immunosuppressive molecule and the nucleic acid sequence encoding the functional exogenous receptor (for example, chimeric antigen receptor, chimeric T cell receptor, etc.) can be located in the same vector or on different vectors.

As used herein, the term "vector" is an intermediary nucleic acid molecule used to transfer (exogenous) genetic material into a host cell, and in the host cell the nucleic acid molecule can be, for example, replicated and/or expressed. Suitable vectors that can be used in the present invention are known in the art, and many are commercially available. In an embodiment, the vector of the present invention includes, but is not limited to, plasmids, virus (for example, retrovirus, adenovirus, adeno-associated virus, vaccinia virus, vesicular stomatitis virus, measles virus, mumps virus, poliovirus, orthomyxovirus, parvovirus, Maraba virus, coxsackie virus, herpes virus, and lentivirus, etc.), phage, phagemid, cosmid, and artificial chromosome (including BAC and YAC). The vector typically also comprises an origin of autonomous replication in the cell (if stable expression of polynucleotide is desired), a selectable marker, and a restriction enzyme cleavage site (e.g., a multiple cloning site, MCS). The vector may additionally comprise elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an in vitro transcription vector.

### Engineered cells

The present invention further provides an engineered cell that expresses the immunosuppressive molecule, the nucleic acid encoding the immunosuppressive molecule, or the vector comprising the nucleic acid of the present invention. In an embodiment, the engineered cell of the present invention further expresses a functional exogenous receptor (as defined below).

In an embodiment, the engineered cell of the present invention is an engineered immune cell.

As used herein, the term "immune cell" refers to any cell of the immune system that has one or more effector functions (for example, cytotoxic cell killing activity, secretion of cytokines, induction of ADCC and/or CDC). For example, the immune cell may be a B cell, a T cell, a macrophage, a dendritic cell, a monocyte, an NK cell, or an NKT cell. The immune cell may be obtained from a variety of sources, for example from a subject (for example, isolated from peripheral blood monocytes, bone marrow, lymph node tissue, cord blood, thymus tissue, tissue from sites of infection, ascites, pleural effusion, spleen tissue, tumors, etc. of the subject), or from a cell line cultured in vitro (for example, Jurkat, SupT1, NK92, etc.), or differentiated from stem cells (for example, derived from cord blood stem cells, progenitor cells, bone marrow stem cells, hematopoietic stem cells, adult stem cells, embryonic stem cells, pluripotent stem cells, iPSCs, etc.). Preferably, the immune cell is a T cell or an NK cell, more preferably a T cell. The T cell also may be concentrated or purified. The T cell may be at any stage of development including, but not limited to, a CD4+CD8+ T cell, a CD4+ T cell (for example, Th1 and Th2 cells), CD8+ T cell (for example, cytotoxic T cell), a CD4-CD8- T cell, a tumor infiltrating cell, a memory T cell, a naive T cell, a γδ-T cell, an αβ-T cell, etc. In a preferred embodiment, the immune cell is a human T cell. The T cell may be isolated from the blood of a subject using a variety of techniques known to those of skill in the art, e.g., Ficoll.

In an embodiment, the expression of at least one of the endogenous HLA class I gene of the engineered cell of the present invention is suppressed or silenced. In an embodiment, the expression of at least one of the endogenous HLA class II gene of the engineered cell of the present invention is suppressed or silenced. In an embodiment, the expression of at least one of the endogenous TCR/CD3 gene of the engineered cell of the present invention is suppressed or silenced. In an embodiment, the expression of at least one of the endogenous TCR/CD3 gene and at least one of the endogenous HLA class I gene of the engineered cell of the present invention is suppressed or silenced. In an embodiment, the expression of at least one of the endogenous TCR/CD3 gene, at least one of the endogenous HLA class I gene and at least one of the endogenous HLA class II gene of the engineered cell of the present invention is suppressed or silenced. Preferably, the HLA class I gene is selected from HLA-A, HLA-B, HLA-C and B2M. Preferably, the HLA class II gene is selected from HLA-DPA, HLA-DQ, HLA-DRA, TAP1, TAP2, LMP2, LMP7, RFX5, RFXAP, RFXANK and CIITA, preferably selected from RFX5, RFXAP, RFXANK and CIITA. Preferably, the TCR/CD3 gene is selected from TRAC, TRBC, CD3γ, CD3δ, CD3ε and CD3ζ.

In an embodiment, expression of one or more endogenous genes of the engineered cell of the present invention is suppressed or silenced, the endogenous genes selected from the group consisting of: CD52, GR, dCK and immune checkpoint genes, e.g., PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

Methods for suppressing gene expression or silencing gene are well known to those skilled in the art, including but not limited to, for example, mediating DNA or RNA fragmentation by meganucleases, zinc finger nucleases, TALEN, CRISPR/Cas system, base editors, or inactivating genes through technologies, e.g., antisense oligonucleotides, RNAi, shRNA, transposons, mutaions, etc.

In an embodiment, the engineered cell of the present invention is an allogeneic cell. As used herein, the term "allogeneic" refers to any material derived from a different animal or a different patient of the same species as the individual into whom the material is introduced. Two or more individuals are considered allogeneic to each other when the genes at one or more loci differ. In some cases, allogeneic material from individuals of the same species may be genetically different enough for antigenic interactions to occur.

### Functional exogenous receptors

n an embodiment, the engineered cell expressing the immunosuppressive molecule of the present invention can further express a functional exogenous receptor. Preferably, the functional exogenous receptor is selected from a recombinant T cell receptor, a chimeric antigen receptor, a T cell fusion protein or a T cell antigen coupler, more preferably a chimeric antigen receptor.

As used herein, the term "T cell fusion protein" or "TFP" refers to a recombinant polypeptide derived from each component of a TCR, usually consisting of a TCR subunit and an antibody linked thereto, and expressed on the cell surface. Wherein, the TCR subunit includes at least part of the TCR extracellular domain, the transmembrane domain, and the TCR intracellular signal domain.

As used herein, the term "T cell antigen coupler" or "TAC" includes three functional domains: 1 a tumor targeting domain, including a single chain antibody (for example, the anti-FasL antibody, the anti-IRP60 antibody, or the anti-SIRPα antibody of the present invention), a designed ankyrin repeat protein (DARPin) or other targeting groups; 2 an extracellular region domain, a CD3-binding single-chain antibody, so that TAC receptors and TCR receptors are close to each other; 3 a transmembrane region and the intracellular region of a CD4 co-receptor, wherein the intracellular region is linked to protein kinase LCK, to catalyze the phosphorylation of the immunoreceptor tyrosine activation motif (ITAM) of the TCR complex as an initial step in T cell activation.

As used herein, the term "T cell receptor" or "TCR" is a characteristic marker on the surface of T cells that binds to CD3 in a non-covalent bond to form a complex. Antigen presenting cells present antigenic peptides to T cells through major histocompatibility complex molecules (MHC) and bind to TCR complexes to induce a series of intracellular signaling. TCR is composed of six peptide chains that form heterodimers, which are generally divided into αβ type and γδ type. Each peptide chain includes a constant region and a variable region, wherein the variable region is responsible for binding specific antigen and MHC molecules. The term "recombinant TCR receptor" refers to an artificially constructed T cell receptor that further comprises an antigen (for example, a tumor antigen) binding domain.

As used herein, the term "chimeric antigen receptor" or "CAR" refers to an artificially constructed hybrid polypeptide that generally includes an antigen (for example, a tumor antigen) binding domain (for example, an antibody or a ligand of antigen), a transmembrane domain, an optional co-stimulatory domain, and a primary signaling domain, and each domain is linked by a linker. CARs are able to redirection the specificity and reactivity of T cells and other immune cells to a selected target in a non-MHC-restricted manner. In an embodiment, the functional exogenous receptor of the present invention is a chimeric antigen receptor, which comprises a tumor antigen binding domain, a transmembrane domain, one or more co-stimulatory domains, and a primary signaling domain. In an embodiment, the chimeric antigen receptor further comprises one or more of the following structures: a signal peptide, a hinge region, a suicide gene, a switch structure, etc.

In an embodiment, the functional exogenous receptor comprises an extracellular domain that specifically recognizes an antigen (for example, a tumor antigen). In an embodiment, the extracellular domain comprises an antibody that specifically binds an antigen or a ligand of the antigen. In an embodiment, the antigen is selected from the group consisting of: ALK, ADRB3, AKAP-4, APRIL, ASGPR1, BCMA, B7H3, B7H4, B7H6, bcr-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CLEC12A, CDH16, CSPG4, CS1, CLL-1, Claudin 6, Claudin18.1, Claudin 18.2, CEA, CEACAM6, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gp130, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region, or any combination thereof. Preferably, the antigen is selected from CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin18.2, ROR1, EGFRvIII, CS1, BCMA and GPRC5D, more preferably selected from CD19, Claudin18.2, MSLN, GPRC5D, CD7 and BCMA.

In an embodiment, the functional exogenous receptor comprises an extracellular domain that specifically recognizes CD19, for example, a antigen targeting CD19. In an embodiment, the antibody targeting CD19 comprises a light chain variable region and a heavy chain variable region, wherein CDR1-L comprised in the light chain variable region is as set forth in SEQ ID NO: 35, CDR2-L is as set forth in SEQ ID NO: 36, and CDR3-L is as set forth in SEQ ID NO: 37; CDR1-H comprised in the heavy chain variable region is as set forth in SEQ ID NO: 32, CDR2-H is set forth in SEQ ID NO: 33, and CDR3-H is as set forth in SEQ ID NO: 34.

In an embodiment, the antibody targeting CD19 comprises a light chain variable region and a heavy chain variable region, wherein the light chain variable region has at least 90%, at least 91%, at least 92%, at least 93%, at least 94%, at least 95%, at least 96%, at least 97%, at least 98%, at least 99%, or 100% identity to SEQ ID NO: 39, 41 or 43, or has modifications of one or more amino acids (for example, at most 1, 2, 3, 4, 5, 6, 7, 8, 9 or 10 amino acids) compared to the amino acid sequence as set forth in SEQ ID NO: 39, 41 or 43; wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 38, 40 or 42, or has modifications of one or more amino acids (for example, at most 1, 2, 3, 4, 5, 6, 7, 8, 9 or 10 amino acids) compared to the amino acid sequence as set forth inSEQ ID NO: 38, 40 or 42. Preferably, the modifications are conservative modifications, for example, conservative substitutions, additions and deletions of amino acids. In a preferred embodiment, the antibody of the present invention comprises a heavy chain variable region as set forth in SEQ ID NO: 38, 40 or 42 and a light chain variable region as set forth in SEQ ID NO: 39, 41 or 43.

In an embodiment, the antibody targeting CD19 has at least 90%, at least 91%, at least 92%, at least 93%, at least 94%, at least 95%, at least 96%, at least 97%, at least 98%, at least 99%, or 100% identity to SEQ ID NO: 44, 45, 46, 47 or 48, or has modifications of one or more amino acids (for example, at most 1, 2, 3, 4, 5, 6, 7, 8, 9 or 10 amino acids) compared to the amino acid sequence as set forth in SEQ ID NO: 44, 45, 46, 47 or 48. Preferably, the antibody targeting FasL is as set forth in SEQ ID NO: 44, 45, 46, 47 or 48.

In an embodiment, the functional exogenous receptor of the present invention is a chimeric antigen receptor, which comprises an antigen binding domain, a transmembrane domain, one or more co-stimulatory domains, and a primary signaling domain. In an embodiment, the functional exogenous receptor of the present invention is a chimeric antigen receptor, the chimeric antigen receptor targets CD19, CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin18.2, ROR1, EGFRvIII, CS1, BCMA or GPRC5D, more preferably targets CD19, Claudin18.2, MSLN, ROR1, GPRC5D, CD7 and BCMA. In an embodiment, the chimeric antigen receptor further comprises a signal peptide, a hinge region, or both. For the definitions of the transmembrane domain, co-stimulatory domain, primary signaling domain, and the optional hinge region, signal peptide and other structures that can be used in the CAR of the present invention are defined in above "immunosuppressive molecules" section.

In an embodiment, the CAR of the present invention may further comprise a switch structure to regulate the expression time of the CAR. For example, the switch structure may be in the form of a dimerization domain, which undergoes a conformational change by binding to its corresponding ligand, exposing the extracellular binding domain, allowing it to bind to the targeted antigen, thereby activating the signal transduction pathway. Alternatively, switch domains may also be used to link the binding domain and the signaling domain, respectively, and only when the switch domains are coupled with each other (for example, in the presence of an inducing compound), the binding domain and the signaling domain can be linked together through the dimerization, thereby activating the signaling pathway. The switch structure may further be in the form of a masking peptide. The masking peptide can mask the extracellular binding region and prevent it from binding to the targeted antigen. When the masking peptide is cleaved by, for example, protease, the extracellular binding region can be exposed, making it a "normal" CAR structure. Various switch structures known to those skilled in the art can be used in the present invention.

In an embodiment, the CAR of the present invention may further comprise a suicide gene, i.e., to make it express a cell death signal that can be induced by an exogenous substance, so as to eliminate CAR cells when needed (for example, when severe toxic side effects occur). For example, the suicide gene may be in the form of inserted epitopes, for example, CD20 epitopes, RQR8, etc., and when necessary, CAR cells can be eliminated by adding antibodies or reagents targeting these epitopes. The suicide gene may also be herpes simplex virus thymidine kinase (HSV-TK), which causes cell death induced by ganciclovir treatment. The suicide gene may further be iCaspase-9, and the dimerization of iCaspase-9 can be induced by a chemically inducing medicament, e.g., AP1903, AP20187, etc., thereby activating the downstream Caspase3 molecule and leading to cell apoptosis. Various suicide genes known to those skilled in the art may be used in the present invention.

### Pharmaceutical composition

The present invention further provides a pharmaceutical composition, which comprises the immunosuppressive molecule, the engineered cell, the nucleic acid molecule or vector of the present invention as an active agent, and one or more pharmaceutically acceptable excipients. Therefore, the present invention further encompasses use of the immunosuppressive molecule, the nucleic acid molecule, the vector or the engineered cell in the preparation of a pharmaceutical composition or a medicament.

As used herein, the term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, and it is well known in the art (see, for example, Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present invention. Exemplary excipients for use in the pharmaceutical composition of the present invention include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present invention is suitable for multiple routes of administration. Generally, the administration is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present invention also may be prepared in various forms, e.g., solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present invention for producing a medicament may include, for example, conventional mixing, dissolving, granulating, sugar-coating, grinding, emulsifying, encapsulating, embedding or lyophilizing process. The pharmaceutical composition comprising, for example, the immune cell as described herein is generally provided in a form of solution, and preferably comprises a pharmaceutically acceptable buffer agent.

The pharmaceutical composition according to the present invention further may be administered in combination with one or more other agents (biological agents, e.g., antibody reagents, and/or small molecules) or treatment methods (for example, surgery, chemotherapy or radiotherapy) suitable for the treatment and/or prevention of diseases to be treated. Preferred examples of agent suitable for the combination include known anti-cancer medicaments, e.g., cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, e.g., ricin, diphtheria toxin, pseudomonas exotoxin A, DNase and RNase; radionuclides, e.g., iodine 131, rhenium 186, indium 111, iridium 90, bismuth 210, bismuth 213, actinides 225 and astatine 213; prodrugs, e.g., antibody-directed enzyme prodrugs; immunostimulatory agents, e.g., platelet factor 4, melanoma growth stimulating protein, etc.; antibodies or fragments thereof, e.g., anti-CD3 antibodies or fragments thereof, complement activators, heterologous protein domains, homologous protein domains, viral/bacterial protein domains and viral/bacterial peptides. In addition, the pharmaceutical composition of the present invention also can be used in combination with one or more other treatment methods, for example, chemotherapy and radiotherapy.

In an embodiment, the pharmaceutical composition of the present invention is used for the treatment of a subject with a cancer, an infection or an autoimmune disease.

In an embodiment, the cancer is a cancer associated with the expression of a target binding to a functional exogenous receptor. For example, the cancer includes, but is not limited to, brain glioma, blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of the digestive system, endometrial cancer, esophageal cancer, eye cancer, head and neck cancer, gastric cancer (including gastrointestinal cancer), glioblastoma (GBM), liver cancer, hepatoma, intraepithelial neoplasia, kidney cancer, laryngeal cancer, liver tumor, lung cancer (for example, small cell lung cancer, non-small cell lung cancer, adenocarcinoma of the lung, and squamous cell lung cancer), lymphoma (including Hodgkin's lymphoma and non-Hodgkin's lymphoma), melanoma, myeloma, neuroblastoma, oral cancer (for example, lip, tongue, mouth, and pharynx), ovarian cancer, pancreatic cancer, prostate cancer, retinoblastoma, rhabdomyosarcoma, colorectal cancer, cancer of the respiratory system, salivary gland cancer, skin cancer, squamous cell carcinoma, gastric cancer, testicular cancer, thyroid cancer, uterine or endometrial cancer, malignancy of the urinary system, vulvar cancer, and other cancers and sarcomas, and B-cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, large mass disease NHL), mantle cell lymphoma, AIDS-related lymphoma, and Waldenstrom macroglobulinemia, chronic lymphocytic leukemia (CLL), acute lymphoblastic leukemia (ALL), B-cell acute lymphoblastic leukemia (B-ALL), T-cell acute lymphoblastic leukemia (T-ALL), B-cell prolymphocytic leukemia, blastic plasmacytoid dendritic cell neoplasm, Burkitt's lymphoma, diffuse large B-cell lymphoma, follicular lymphoma, chronic myeloid leukemia (CML), malignant lymphoproliferative disease, MALT lymphoma, hairy cell leukemia, marginal zone lymphoma, multiple myeloma, myelodysplasia, plasmablastic lymphoma, preleukemia, plasmacytoid dendritic cell neoplasm, and post-transplant lymphoproliferative disorder (PTLD); and other diseases associated with target expression. Preferably, the disease that can be treated with the engineered immune cell or the pharmaceutical composition of the present invention is selected from the group consisting of: leukemia, lymphoma, multiple myeloma, brain glioma, pancreatic cancer, gastric cancer, etc.

In an embodiment, the infection includes, but is not limited to, infections caused by viruses, bacteria, fungi, and parasites.

In an embodiment, the autoimmune disease includes, but is not limited to type I diabetes, celiac disease, Graves' disease, inflammatory bowel disease, multiple sclerosis, psoriasis, rheumatoid arthritis, Addison's disease, Sjögren's syndrome, Hashimoto's thyroiditis, myasthenia gravis, vasculitis, pernicious anemia and systemic lupus erythematosus, etc.

### Method for suppressing immune rejection of exogenous cells by immune cells of a subject

In one aspect, the present invention further provides a method for reducing immune rejection of exogenous cells by cells of a subject, characterized by expressing the immunosuppressive molecule, the nucleic acid molecule encoding the immunosuppressive molecule or the vector comprising the nucleic acid molecule of the present invention in the exogenous cells.

In another aspect, the present invention further provides a method for reducing immune rejection of exogenous cells by cells of a subject, characterized by administering the immunosuppressive molecule or the engineered cell expressing the immunosuppressive molecule of the present invention to the subject. In an embodiment, the immunosuppressive molecule or the engineered cell of the present invention is administered simultaneously or sequentially with the exogenous cells. For example, the immunosuppressive molecule or the engineered cell of the present invention can be administered after the exogenous cells.

As used herein, the term "subject" is a mammal. Mammals can be humans, non-human primates, mice, rats, dogs, cats, pigs, horses, or cows, but are not limited to these examples. Mammals other than humans can be advantageously used as subjects representing animal models of cancer. Preferably, the subject is a human.

The present invention will be described in detail below with reference to the accompanying drawings and examples. It should be noted that those skilled in the art should understand that the drawings of the present invention and the examples thereof are only for the purpose of illustration, and shall not constitute any limitation to the present invention. In the case of no contradiction, the examples in the present application and the features in the examples can be combined with each other.

### BRIEF DESCRIPTION OF DRAWINGS

Figure 1 shows the CAR expression level of CAR-T cells expressing the immunosuppressive molecule targeting FasL.
Figure 2 shows the killing effect of CAR-T cells expressing the immunosuppressive molecule targeting FasL on target cells.
Figure 3 shows the cytokine release level of CAR-T cells expressing the immunosuppressive molecule targeting FasL after co-incubation with target cells Nalm6.
Figure 4 shows the cytokine release level of CAR-T cells expressing the immunosuppressive molecule targeting FasL after co-incubation with target cells Raji.
Figure 5 shows the suppressive effect of the immunosuppressive molecule targeting FasL on the proliferation of allogeneic PBMC.
Figure 6 shows the in vivo tumor suppression effect of CAR-T cells expressing the immunosuppressive molecule targeting FasL.
Figure 7 shows the CAR expression level of CAR-T cells expressing the immunosuppressive molecule targeting SIRPα.
Figure 8 shows the expression level of anti-SIRPα antibody in CAR-T cells expressing the immunosuppressive molecule targeting SIRPα.
Figure 9 shows the cytokine release level of CAR-T cells expressing the immunosuppressive molecule targeting SIRPα after co-incubation with target cells Nalm6.
Figure 10 shows the cytokine release level of CAR-T cells expressing the immunosuppressive molecule targeting SIRPα after co-incubation with target cells Raji.
Figure 11 shows the suppressive effect of the immunosuppressive molecule targeting SIRPα on NK cell killing.
Figure 12 shows the CAR expression level of CAR-T cells expressing the immunosuppressive molecule targeting IRP60.
Figure 13 shows the expression level of anti-IRP60 antibody in CAR-T cells expressing the immunosuppressive molecule targeting IRP60.
Figure 14 shows the cytokine release level of CAR-T cells expressing the immunosuppressive molecule targeting IRP60 after co-incubation with target cells Nalm6.
Figure 15 shows the cytokine release level of CAR-T cells expressing the immunosuppressive molecule targeting IRP60 after co-incubation with target cells Raji.
Figure 16 shows the suppressive effect of the immunosuppressive molecule targeting IRP60 on NK cell killing.
Figure 17 shows the CAR expression level of CAR-T cells expressing the immunosuppressive molecule comprising CD47.
Figure 18 shows the expression level of CD47 in CAR-T cells expressing the immunosuppressive molecule comprising CD47.
Figure 19 shows the killing effect of CAR-T cells expressing the immunosuppressive molecule comprising CD47 on target cells Raji.
Figure 20 shows the cytokine release level of CAR-T cells expressing the immunosuppressive molecule comprising CD47 after co-incubation with target cells Raji.
Figure 21 shows the suppressive effect of the immunosuppressive molecule comprising CD47 on NK cell killing.

### Examples

### Example 1. Construction of CAR-T immune cells expressing the immunosuppressive molecule

Sequences encoding the following were synthesized and sequentially cloned into the pGEM-T Easy vector (Promega): anti-CD19 scFv (SEQ ID NO: 44), CD8α hinge region (SEQ ID NO: 29), CD8α transmembrane region (SEQ ID NO: 17), 4-1BB co-stimulatory domain (SEQ ID NO: 20), CD3ζ intracellular region (SEQ ID NO: 23) to obtain CAR19 plasmid, and the correct insertion of the target sequence in the plasmid was confirmed by sequencing.

The immunosuppressive molecule linked by T2A (SEQ ID NO: 31) was further expressed in the above CAR19 plasmid, and its structure was as follows: B2m signal peptide (SEQ ID NO: 24), anti-FasL scFv-1 (SEQ ID NO: 9) or anti-FasL scFv-2 (SEQ ID NO: 14), IgG4 hinge region (SEQ ID NO: 30), CD28 transmembrane region (SEQ ID NO: 15), CD28 co-stimulatory domain (SEQ ID NO: 18) to obtain CAR19-aFL-1 plasmid and CAR19-aFL-2 plasmid, and the correct insertion of the target sequence in the plasmid was confirmed by sequencing.

The following structures linked by T2A (SEQ ID NO: 31) were further expressed in the above CAR19 plasmid: B2m signal peptide (SEQ ID NO: 24), Fas extracellular region and transmembrane region (SEQ ID NO: 49), IgG4 hinge region (SEQ ID NO: 30), CD28 transmembrane region (SEQ ID NO: 15), CD28 co-stimulatory domain (SEQ ID NO: 18) to obtain CAR19-Fas plasmid, and the correct insertion of the target sequence in the plasmid was confirmed by sequencing.

Three ml Opti-MEM (Gibco) was added to a sterile tube to dilute the above plasmid, and then packaging vector psPAX2 (Addgene) and envelope vector pMD2.G (Addgene) were added according to the ratio of plasmid : viral packaging vector : viral envelope vector = 4:2:1. Then, 120 µl X-treme GENE HP DNA transfection reagent (Roche) was added, mixed immediately, and incubated at room temperature for 15 min, and then the plasmid/vector/transfection reagent mixture was added dropwise to the culture flask of 293T cells. Viruses were collected at 24 hours and 48 hours, pooled, and ultracentrifuged (25000 g, 4°C, 2.5 hours) to obtain concentrated lentiviruses.

T cells were activated with DynaBeads CD3/CD28 CTSTM (Gibco), and were further cultured for 1 day at 37°C and 5% CO₂. On the second day, the concentrated lentiviruses were added, and after 3 days of continuous culture, T cells expressing CD19 CAR (CAR19-T) and T cells expressing a combination of CD19 CAR + immunosuppressive molecule targeting FasL (CAR19-Fas T, CAR19-aFL-1 T, and CAR19-aFL-2 T) were obtained.

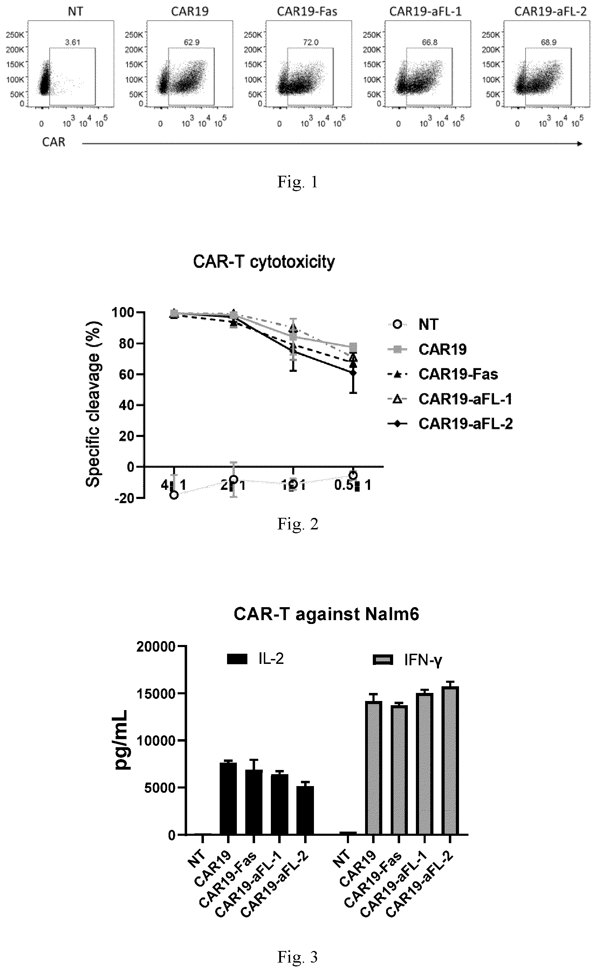

In addition, using a flow cytometry, the expression of anti-CD19 scFv in CAR-T cells was detected with FITC goat anti-human Fab (Thermo Fisher). It can be seen that all the CARs in the CAR-T cells prepared in this example can be effectively expressed (Figure 1).

### Example 2. Killing effect of CAR-T cells on target cells and cytokine release level

Target cells Raji cells, were plated into 96-well plates at a concentration of 1×10⁴ cells/well, and then, NT cells, CAR19 T cells, CAR19-Fas T cells, CAR19-aFL-1 T cells and CAR19-aFL-2 T cells were plated into the 96-well plates at an effector-to-target ratio of 4:1, 2:1, 1:1 or 0.5:1 for co-culture. After 18 hours, the fluorescence value was measured with a microplate reader. According to the calculation formula: (average fluorescence value of target cells - average fluorescence value of samples)/average fluorescence value of target cells × 100%, the killing efficiency was calculated, and the results are shown in Figure 2.

It can be seen that CAR19-Fas T cells, CAR19-aFL-1 T cells and CAR19-aFL-2 T cells showed effective killing on target cells, and the killing effect thereof was slightly better than that of CAR19 T cells that did not express the immunosuppressive molecule at a low effector-to-target ratio (for example, 0.5:1).

In addition, target cells (Nalm6 cells and Raji cells) were plated in 96-well plates at a concentration of 1×10⁵ cells/well, NT cells, CAR19 T cells, CAR19-Fas T cells, CAR19-aFL-1 T cells, and CAR19-aFL-2 T cells were added at a ratio of 1:1, respectively, and the cell co-culture supernatant was collected after 18-24 hours of co-culture.

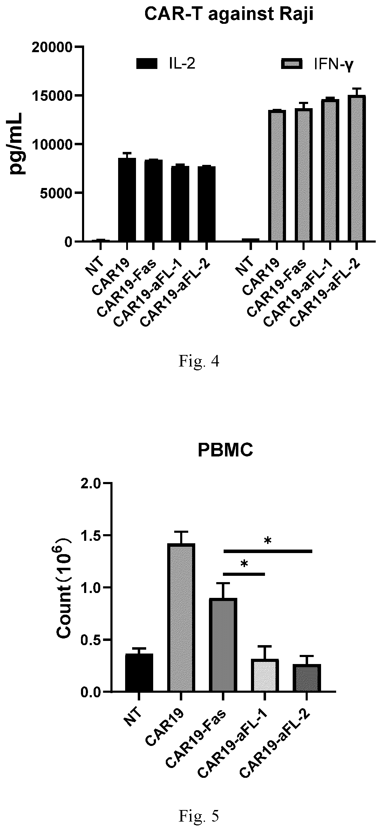

Human IL-2 DuoSet ELISA Kit (R&D systems) and Human IFN-gamma DuoSet ELISA Kit (R&D systems) were used to detect the contents of IL2 and IFN-γ in the co-culture supernatant, respectively, and the results are shown in Figure 3 and Figure 4. It can be seen that compared with NT cells, after co-culture with target cells, the release levels of cytokines IL2 and IFN-γ of various CAR-T cells were significantly increased, indicating that this cytokine release was specific. In addition, the cytokine release levels of various CAR-T cells were basically the same.

These data indicated that additional expression of the immunosuppressive molecule targeting FasL does not change the cytokine release level of CAR-T cells, and slightly improves the in vitro killing activity of CAR-T cells at a low effector-to-target ratio.

### Example 3. In vitro HvGD

The suppressive effect of the immunosuppressive molecule of the present invention on HvGD in vitro was assessed by one-way mixed lymphocyte reaction (MLR) assay. Specifically, NT cells, CAR19 T cells, CAR19-Fas T cells, CAR19-aFL-1 T cells and CAR19-aFL-2 T cells were co-incubated with allogeneic PBMCs for 7 days at 37°C and 5% CO₂, and then the proliferation of PBMCs was detected by flow cytometry, and the results are shown in Figure 5.

It can be seen that compared with NT cells, CAR19 T cells increased the count of PBMCs by about 3.5-fold (0.4x10⁶ vs 1.4x10⁶), which indicates that heterologous CAR-T cells greatly promoted the proliferation of PBMCs. In addition, it was also noted that additional expression of the immunosuppressive molecule can significantly reduce this proliferation of PBMCs. In particular, it is worth noting that the immunosuppressive molecule comprising Fas extracellular region (CAR19-Fas T) reduced PBMC proliferation by about 35% (about 0.9x10⁶), while the immunosuppressive molecule comprising anti-FasL scFv (CAR19-aFL-1 T and CAR19-aFL-2 T) further reduced PBMC proliferation by about 3-fold (about 0.3x10⁶) on the basis of CAR19-Fas T cells, which is basically comparable to the effect of NT cells that do not express CARs.

In summary, the additional expression of the immunosuppressive molecule targeting FasL can significantly suppress the in vitro proliferation of allogeneic PBMCs, which in turn significantly reduces HvGD. Moreover, such suppressive effect of the immunosuppressive molecule comprising anti-FasL scFv is significantly better than that of the immunosuppressive molecule comprising Fas extracellular region.

### Example 4. In vivo suppressive effect of CAR T cells on tumors

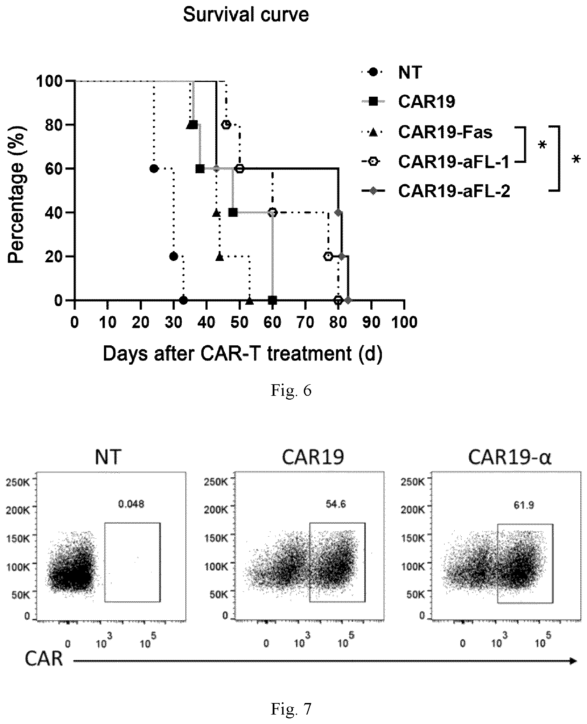

Twenty-five healthy female NPI mice aged 6-8 weeks were divided into 5 groups, with 5 mice in each group: NT group, CAR19 group, CAR19-Fas group, CAR19-aFL-1 group, and CAR19-aFL-2 group. On day 0, 5×10⁵ Raji cells were injected into the tail vein of each mouse. Six days later, 2x10⁶ NT cells or corresponding CAR-T cells were injected into the tail vein of each mouse according to the grouping. The status of the mice was assessed weekly. The mouse survival curve is shown in Figure 6.

It can be seen that the survival rate of CAR19-Fas group is comparable to that of CAR19 group, indicating that the immunosuppressive molecule comprising Fas extracellular region may not exert a good suppressive effect on immune rejection in vivo. On the contrary, the survival rates of CAR19-aFL-1 group and CAR19-aFL-2 group were significantly higher than that of CAR19-Fas group, indicating that the immunosuppressive molecule comprising anti-FasL scFv can also effectively suprpress immune rejection in vivo, which in turn enhances the tumor suppression effect of CAR-T cells and improves the survival rate.

### Example 5. Construction of CAR-T cells expressing the immunosuppressive molecule targeting SIRPα and verification of the function thereof

### 5.1 Construction of CAR-T cells

CAR-T cells expressing the immunosuppressive molecule targeting SIRPα were constructed according to the method of Example 1, wherein the B2M gene in T cells was knocked out using the CRISPR/Cas9 system before the lentiviral transfection step. The immunosuppressive molecule targeting SIRPα comprises: B2m signal peptide (SEQ ID NO: 24), anti-SIRPα scFv (SEQ ID NO: 58), IgG4 hinge region (SEQ ID NO: 30), CD28 transmembrane region (SEQ ID NO: 15), and CD28 co-stimulatory domain (SEQ ID NO: 18). The obtained CAR-T cells were named CAR19-α T cells.

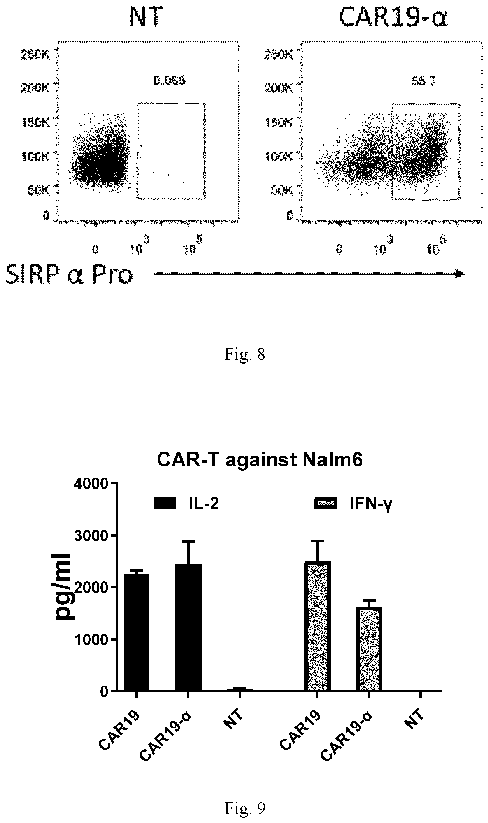

Using a flow cytometry, the expression of anti-CD19 scFv in CAR-T cells was detected with FITC goat anti-human Fab (Thermo Fisher), and the expression of anti-SIRPα scFv was detected with Human SIRPα protein (Acro). It can be seen that all the CARs in the CAR-T cells prepared in this example can be effectively expressed (Figure 7), and the immunosuppressive molecule in CAR19-α T cells can also be effectively expressed (Figure 8).

### 5.2 Detection of the cytokine release level after co-incubation with target cells

Target cells (Nalm6 cells and Raji cells) were plated in 96-well plates at a concentration of 1×10⁵ cells/well, CAR19-T cells, CAR19-α T cells and NT cells (negative control) were added at a ratio of 1:1, respectively, and the cell co-culture supernatant was collected after 18-24 hours of co-culture.

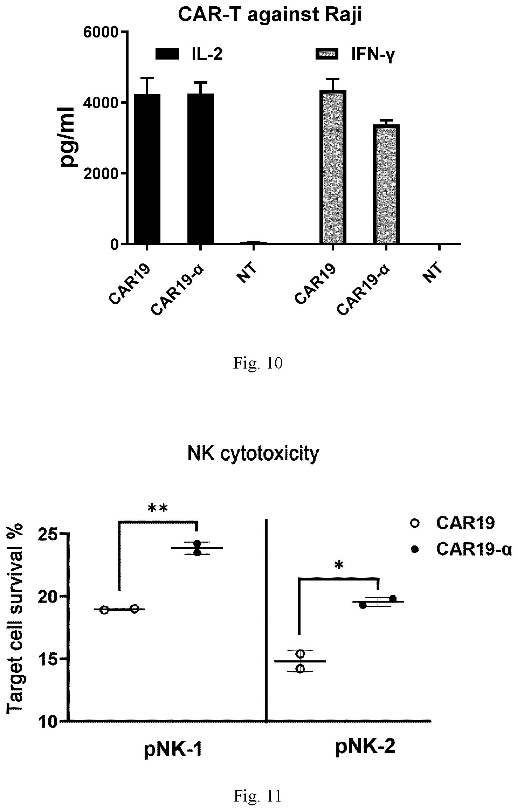

Human IL-2 DuoSet ELISA Kit (R&D systems) and Human IFN-gamma DuoSet ELISA Kit (R&D systems) were used to detect the contents of IL2 and IFN-γ in the co-culture supernatant, respectively, and the results are shown in Figure 9 and Figure 10. It can be seen that compared with NT cells, after co-culture with each target cell, the release levels of cytokines IL2 and IFN-γ of CAR19-T and CAR19-α T were significantly increased, indicating that this cytokine release was specific. In addition, the cytokine release levels of CAR19-T and CAR19-α T cells are comparable, indicating that the additionally expressed immunosuppressive molecule does not impair the cytokine release characteristic of CAR-T cells.

### 5.3. Detection of the suppressive effect on NK cell killing

The CAR-T cells prepared in this example were labeled with Far-Red (invitrogen). The labeled CAR-T cells were then plated into 96-well plates at a concentration of 1x10⁴ cells/well, and allogeneic NK cells (pNK-1 and pNK-2) derived from peripheral blood were added at a effector-to-target ratio of 2:1 (NK cells : CAR-T cells) for co-culture. After 16-18 hours, the survival ratio of T cells in the culture was detected using a flow cytometry, and the results are shown in Figure 11.

It can be seen that compared with conventional CAR19-T cells, after co-culture with NK cells, the survival rate of CAR19-α T cells expressing the immunosuppressive molecule is significantly improved. This indicates that the immunosuppressive molecule of the present invention can effectively suppress the killing of NK cells on CAR-T cells expressing the immunosuppressive molecule. This is particularly effective in reducing immune rejection reaction.

### Example 6. Construction of CAR-T cells expressing the immunosuppressive molecule targeting IRP60 and verification of the function thereof

### 6.1 Construction of CAR-T cells

CAR-T cells expressing the immunosuppressive molecule targeting IRP60 were constructed according to the method of Example 1, wherein the B2M gene in T cells was knocked out using the CRISPR/Cas9 system before the lentiviral transfection step. The immunosuppressive molecule targeting IRP60 comprises: B2m signal peptide (SEQ ID NO: 24), anti-IRP60 scFv (SEQ ID NO: 67), IgG4 hinge region (SEQ ID NO: 30), CD28 transmembrane region (SEQ ID NO: 15), and CD28 co-stimulatory domain (SEQ ID NO: 18). The obtained CAR-T cells were named CAR19-60 T cells.

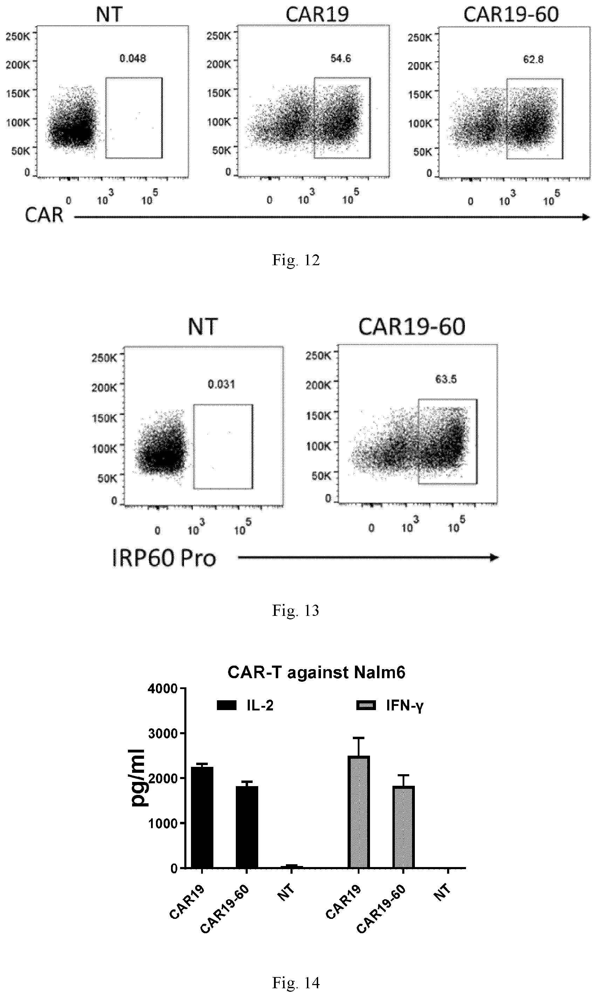

Using a flow cytometry, the expression of anti-CD 19 scFv in CAR-T cells was detected with FITC goat anti-human Fab (Thermo Fisher), and the expression of anti-IRP60 scFv was detected with Human IRP60 protein (Kactus). It can be seen that all the CARs in the CAR-T cells prepared in this example can be effectively expressed (Figure 12), and the immunosuppressive molecule in CAR19-60 T cells can also be effectively expressed (Figure 13).

### 6.2 Detection of the cytokine release level after co-incubation with target cells

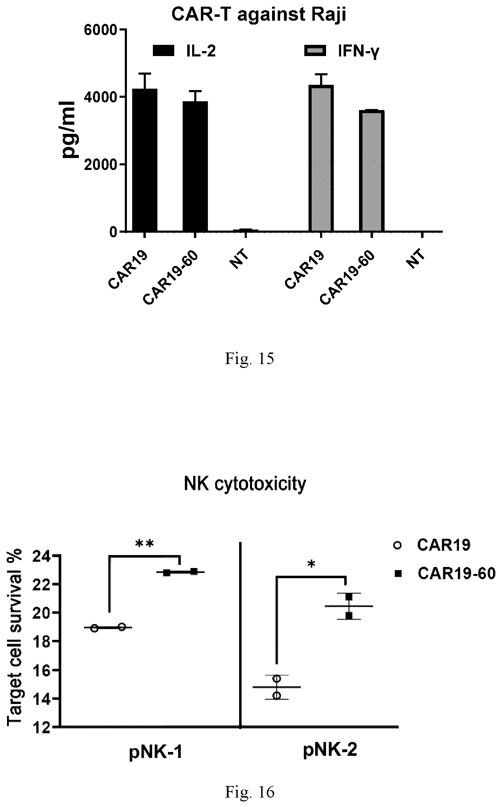

The release levels of cytokines IL2 and IFN-γ of the CAR-T cells prepared in this example after co-incubation of with target cells (Nalm6 cells and Raji cells) were detected according to the method as described in Example 5.2, and the results are shown in Figure 14 and Figure 15. It can be seen that compared with NT cells, after co-culture with each target cell, the release levels of cytokines IL2 and IFN-γ of CAR19-T and CAR19-60 T were significantly increased, indicating that this cytokine release was specific. In addition, the cytokine release levels of CAR19-T and CAR19-60 T cells are comparable, indicating that the additionally expressed immunosuppressive molecule does not impair the cytokine release characteristic of CAR-T cells.

### 6.3. Detection of the suppressive effect on NK cell killing

The suppressive effect of the CAR-T cells prepared in this example on the killing of allogeneic NK cells was detected according to the method as described in Example 5.3, and the results are shown in Figure 16.

It can be seen that compared with conventional CAR19-T cells, after co-culture with NK cells, the survival rate of CAR19-60 T cells expressing the immunosuppressive molecul is significantly improved. This indicates that the immunosuppressive molecule of the present invention can effectively suppress the killing of NK cells on CAR-T cells expressing the immunosuppressive molecule.

### Example 7. Construction of CAR-T immune cells expressing the immunosuppressive molecule comprising CD47 and verification of the function thereof

### 7.1 Construction of CAR-T cells

Sequences encoding the following were synthesized and sequentially cloned into pGEM-T Easy vector (Promega, Cat. No. A1360): anti-CD19 scFv (SEQ ID NO: 44), CD8α hinge region (SEQ ID NO: 29), CD8α transmembrane region (SEQ ID NO: 17), 4-1BB co-stimulatory domain (SEQ ID NO: 20), CD3ζ intracellular region (SEQ ID NO: 23) to obtain CAR19 plasmid, and the correct insertion of the target sequence in the plasmid was confirmed by sequencing.

The immunosuppressive molecule linked by T2A (SEQ ID NO: 31) was further expressed in the above CAR19 plasmid, and its structure was as follows: CD47 extracellular transmembrane region (SEQ ID NO: 68), CD28 co-stimulatory domain (SEQ ID NO: 18) to obtain CAR19-47 plasmid, and the correct insertion of the target sequence in the plasmid was confirmed by sequencing.

Three ml Opti-MEM (Gibco, Cat. No. 31985-070) was added to a sterile tube to dilute the above plasmid, and then packaging vector psPAX2 (Addgene, Cat. No. 12260) and envelope vector pMD2.G (Addgene, Cat. No. 12259) were added according to the ratio of plasmid : viral packaging vector : viral envelope vector = 4:2:1. Then, 120 µl X-treme GENE HP DNA transfection reagent (Roche, Cat. No. 06366236001) was added, mixed immediately, and incubated at room temperature for 15 min, and then the plasmid/vector/transfection reagent mixture was added dropwise to the culture flask of 293T cells. Viruses were collected at 24 hours and 48 hours, pooled, and ultracentrifuged (25000 g, 4°C, 2.5 hours) to obtain concentrated lentiviruses.

T cells were activated with DynaBeads CD3/CD28 CTSTM (Gibco, Cat. No. 40203D), and were cultured for 1 day at 37°C and 5% CO₂. Then, the concentrated lentiviruses were added, and after 3 days of continuous culture, T cells expressing CD19 CAR (CAR19-T) and T cells expressing CD19 CAR and the immunosuppressive molecule targeting SIRPα (CAR19-47-T) were obtained.

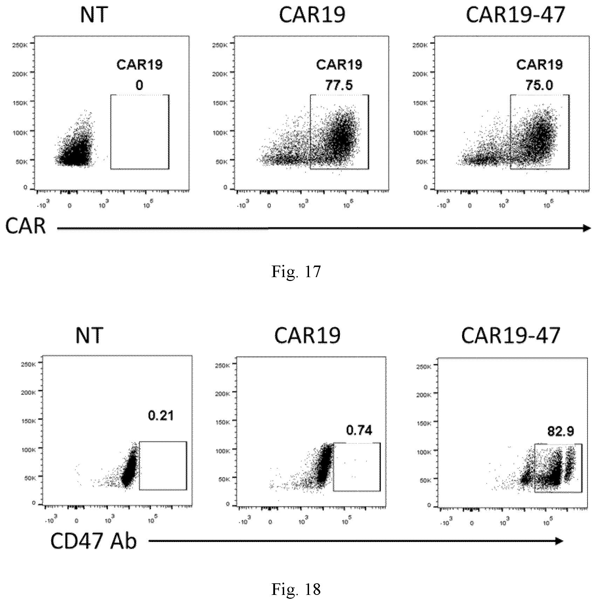

In addition, using a flow cytometry, the expression of anti-CD19 scFv in CAR-T cells was detected with FITC goat anti-human Fab (Thermo Fisher, Cat. No. 31628), and the expression of was detected with mouse anti-human CD47 Ab (Biolegend, Cat. No. 323108). It can be seen that all the CARs in the CAR-T cells prepared by the present invention can be effectively expressed (Figure 17), and the immunosuppressive molecule in CAR19-CD47-T cells can also be effectively expressed (Figure. 18).

### 7.2 Detection of the killing effect of CAR-T cells on target cells

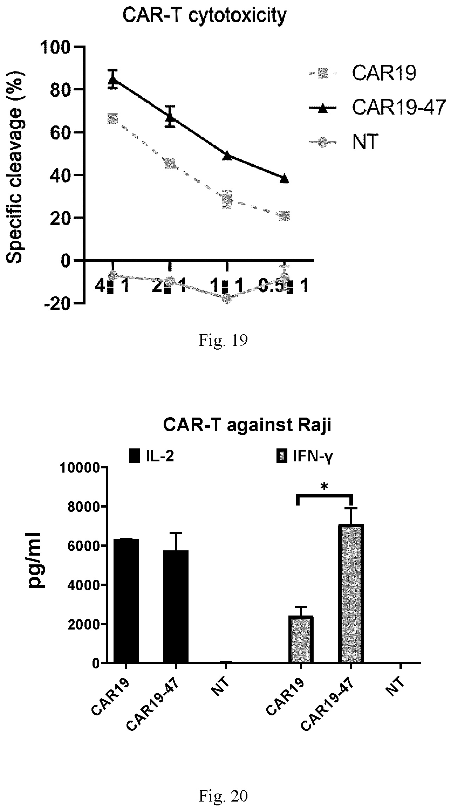

Target cells Raji cells, were plated into 96-well plates at a concentration of 1×10⁴ cells/well, and then, NT cells, CAR19-T T cells and CAR19-47-T cells were plated into the 96-well plates at an effector-to-target ratio of 4:1, 2:1, 1:1 or 0.5:1 for co-culture. After 18 hours, the fluorescence value was measured with a microplate reader. According to the calculation formula: (average fluorescence value of target cells - average fluorescence value of samples)/average fluorescence value of target cells × 100%, the killing efficiency was calculated, and the results are shown in Figure 19.

It can be seen that both CAR19-T cells and CAR19-47-T cells showed effective specific killing of target cells, and compared with CAR19-T, the killing activity of CAR19-47-T cells was significantly improved.

### 7.3. Detection of the cytokine release level of CAR-T cells after co-incubation with target cells

Target cells (Raji cells) were plated in 96-well plates at a concentration of 1×10⁵ cells/well, CAR19-T cells, CAR19-CD47-T cells and NT cells (negative control) were added at a ratio of 1:1, respectively, and the cell co-culture supernatant was collected after 18-24 hours of co-culture.

Human IL-2 DuoSet ELISA Kit (R&D systems, Cat. No. DY202) and Human IFN-gamma DuoSet ELISA Kit (R&D systems, Cat. No. DY285) were used to detect the contents of IL2 and IFN-γ in the co-culture supernatant, respectively, and the results are shown in Figure 20.

It can be seen that compared with NT cells, after co-culture with the target cells, the release levels of cytokines IL2 and IFN-γ of CAR19-T and CAR19-47-T were significantly increased, indicating that this cytokine release was specific. In addition, the secretion capacity of IFN-γ of CAR19-CD47-T was significantly higher than that of CAR19-T cells.

### Example 7.4. Dectection of the suppressive effect of the immunosuppressive molecule on NK cell killing

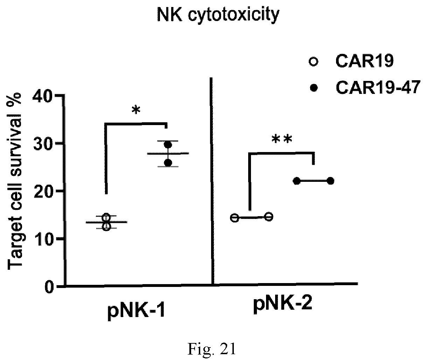

The CAR-T cells prepared by the present invention were labeled with Far-Red (invitrogen, Cat. No. C34564). Then, the labeled CAR-T cells and cells were plated into 96-well plates at a concentration of 1x10⁴ cells/well, and NK cells derived from peripheral blood were added at an effector-to-target ratio of 2:1 for co-culture. After 16-18 hours, the survival ratio of T cells in the culture was detected using a flow cytometry, and the results are shown in Figure 21.

It can be seen that compared with conventional CAR19-T cells, after co-culture with NK cells, the survival rate of CAR19-47 cells expressing the immunosuppressive molecule is significantly improved. This indicates that the immunosuppressive molecule of the present invention can effectively suppress the killing of NK cells on CAR-T cells.

In summary, on the one hand, the immunosuppressive molecule targeting FasL, IRP60 or SIRPα of the present invention will not adversely affect the killing activity or the cytokine release characteristic of CAR-T cells and on the other hand, the immunosuppressive molecule targeting FasL, IRP60 or SIRPα of the present invention can significantly suppress the killing of allogeneic NK cells or suppress the excessive proliferation of allogeneic PBMCs after being activated, thereby effectively reducing immune rejection of exogenous cells by immune cells in the host.

It should be noted that the above-mentioned are merely for preferred examples of the present invention and not used to limit the present invention. For those skilled in the art, various modifications and changes may be made to the present invention. Those skilled in the art should understand that any amendments, equivalent replacements, improvements, and so on, made within the spirit and principle of the present invention, should be covered within the scope of protection of the present invention.

### INDUSTRIAL UTILITY

The present invention relates to an immunosuppressive molecule, comprising an immunosuppressive protein binding domain, a transmembrane domain, and a co-stimulatory domain and not comprising a primary signaling domain, wherein the immunosuppressive protein binding domain is selected from: an antibody targeting FasL, an antibody targeting IRP60, a ligand of IRP60 or a functional fragment thereof, an antibody targeting SIRPa, a ligand of SIRPa or a functional fragment thereof, wherein the immunosuppressive molecule can reduce immune rejection by cells of a subject. The immunosuppressive molecule of the present invention effectively reduces immune rejection of exogenous cells by immune cells in a host, and has excellent industrial utility.

## Claims

1. An immunosuppressive molecule, comprising an immunosuppressive protein binding domain, a transmembrane domain, and a co-stimulatory domain and not comprising a primary signaling domain, wherein the immunosuppressive protein binding domain is selected from: an antibody targeting FasL, an antibody targeting IRP60, a ligand of IRP60 or a functional fragment thereof, an antibody targeting SIRPa, a ligand of SIRPa or a functional fragment thereof, wherein the immunosuppressive molecule can reduce immune rejection by cells of a subject.

2. The immunosuppressive molecule of claim 2, **characterized in that** the antibody is selected from the group consisting of: a whole antibody, Fab, Fab', F(ab')2, an Fv fragment, scFv, a linear antibody, sdAb or a nanobody.

3. The immunosuppressive molecule of any one of claims 1-2, **characterized in that** the transmembrane domain is selected from a transmembrane domain of a protein selected from the group consisting of a TCR α chain, a TCR β chain, a TCR γ chain, a TCR δ chain, a CD3 ζ subunit, a CD3 ε subunit, a CD3 γ subunit, a CD3 δ subunit, CD28, CD45, CD4, CD5, CD8a, CD9, CD16, CD22, CD33, CD37, CD47, CD64, CD80, CD86, CD94, CD134, CD137, CD154, KIRDS2, OX40, CD2, CD27, CD18, ICOS, 4-1BB, GITR, CD40, BAFFR, HVEM, SLAMF7, NKp80, CD160, BCMA, IL-2R β, IL-2R γ, IL-7R a, ITGA1, VLA1, CD49a, ITGA4, IA4, CD49D, ITGA6, VLA-6, CD49f, ITGAD, CDlld, ITGAE, CD103, ITGAL, CDlla, LFA-1, ITGAM, CDllb, ITGAX, CDllc, ITGB1, CD29, ITGB2, CD18, LFA-1, ITGB7, TNFR2, DNAM1, SLAMF4, CD84, CD96, CEACAM1, CRT AM, Ly9, CD160, PSGL1, CDIOO, SLAMF6, SLAMF1, SLAMF8, CD162, LTBR, PAG/Cbp, NKp44, NKp30, NKp46, NKG2D or NKG2C.

4. The immunosuppressive molecule of any one of claims 1-3, **characterized in that** the co-stimulatory domain is selected from an intracellular region of a protein selected from the group consisting of LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, 4-1BB, CD270, CD272, B7-H3, ICOS, CD357, DAP10, DAP12, LAT, NKG2C, SLP76, PD-1, LIGHT, TRIM, ZAP70 and combinations thereof.

5. The immunosuppressive molecule of any one of claims 1-4, **characterized in that** the primary signaling domain is selected from an intracellular region of a protein selected from the group consisting of: FcRγ, FcRβ, CD3γ, CD3δ, CD3ε, CD3ζ, CD5, CD22, CD79a, CD79b, NFAM1, STAM1, STAM2, and Cd66d.

6. The immunosuppressive molecule of any one of claims 1-5, further comprising a signal peptide.

7. The immunosuppressive molecule of any one of claims 1-6, further comprising a hinge region derived from the group consisting of CD8α, CD28, a FcγRIIIα receptor, IgG4 or IgG1.

8. The immunosuppressive molecule of any one of claims 1-7, **characterized in that** the immunosuppressive protein binding domain is an antibody targeting FasL.

9. The immunosuppressive molecule of claim 8, **characterized in that** the antibody targeting FasL comprises a light chain variable region and a heavy chain variable region, wherein CDR1-L, CDR2-L and CDR3-L comprised in the light chain variable region are the same as CDR1-L, CDR2-L and CDR3-L comprised in SEQ ID NO: 8; wherein CDR1-H, CDR2-H and CDR3-H comprised in the heavy chain variable region are the same as CDR1-H, CDR2-H and CDR3-H comprised in SEQ ID NO: 7 or 13;
preferably, the CDR1-L is as set forth in SEQ ID NO: 4, CDR2-L is as set forth in SEQ ID NO: 5, CDR3-L is as set forth in SEQ ID NO: 6, CDR1-H is as set forth in SEQ ID NO: 1 or 10, CDR2-H is as set forth in SEQ ID NO: 2 or 11, and CDR3-H is as set forth in SEQ ID NO: 3 or 12;
preferably, wherein the light chain variable region is as set forth in SEQ ID NO: 8, and wherein the heavy chain variable region is as set forth in SEQ ID NO: 7 or 13;
preferably, wherein the antibody targeting FasL is as set forth in SEQ ID NO: 9 or 14.

10. The immunosuppressive molecule of claim 1, **characterized in that** the immunosuppressive protein binding domain is an antibody targeting SIRPα, a ligand of SIRPα or a functional fragment thereof.

11. The immunosuppressive molecule of claim 10, **characterized in that** the antibody targeting SIRPα comprises a light chain variable region and a heavy chain variable region, wherein CDR1-L, CDR2-L and CDR3-L comprised in the light chain variable region are the same as CDR1-L, CDR2-L and CDR3-L comprised in SEQ ID NO: 57; wherein CDR1-H, CDR2-H and CDR3-H comprised in the heavy chain variable region are the same as the CDR1-H, CDR2-H and CDR3-Hcontained in SEQ ID NO: 56;
preferably, the CDR1-L is as set forth in SEQ ID NO: 53, CDR2-L is as set forth in SEQ ID NO: 54, CDR3-L is as set forth in SEQ ID NO: 55, CDR1-H is as set forth in SEQ ID NO: 50, CDR2-H is as set forth in SEQ ID NO: 51, and CDR3-H is as set forth in SEQ ID NO: 52;
preferably, wherein the light chain variable region is as set forth in SEQ ID NO: 57, and wherein the heavy chain variable region is as set forth in SEQ ID NO: 56;
preferably, the antibody targeting SIRPα is as set forth in SEQ ID NO:58.

12. The immunosuppressive molecule of claim 10, **characterized in that** the ligand of SIRPα is CD47 or its extracellular region;
preferably, wherein the ligand of SIRPα is as set forth in SEQ ID NO: 69;

13. The immunosuppressive molecule of claim 1, **characterized in that** the immunosuppressive protein binding domain is an antibody targeting IRP60, a ligand of IRP60 or a functional fragment thereof.

14. The immunosuppressive molecule of claim 13, **characterized in that** the antibody targeting IRP60 comprises a light chain variable region and a heavy chain variable region, wherein CDR1-L, CDR2-L and CDR3-L comprised in the light chain variable region are the same as CDR1-L, CDR2-L and CDR3-L contained in SEQ ID NO: 66; wherein CDR1-H, CDR2-H and CDR3-H comprised in the heavy chain variable region are the same as CDR1-H, CDR2-H and CDR3-H as comprised in SEQ ID NO: 65,
preferably, the CDR1-L is as set forth in SEQ ID NO: 64, CDR2-L is as set forth in SEQ ID NO: 63, CDR3-L is as set forth in SEQ ID NO: 62, CDR1-H is as set forth in SEQ ID NO: 59, CDR2-H is as set forth in SEQ ID NO: 60, and CDR3-H is as set forth in SEQ ID NO: 61;
preferably, wherein the light chain variable region is as set forth in SEQ ID NO:66, and wherein the heavy chain variable region is as set forth in SEQ ID NO:65;
preferably, wherein the antibody targeting IRP60 is as set forth in SEQ ID NO:67.

15. A nucleic acid molecule encoding the immunosuppressive molecule according to any one of claims 1-14.

16. A vector comprising the nucleic acid according to claim 15.

17. The vector of claim 15, **characterized in that** the vector is selected from the group consisting of retrovirus, adenovirus, adeno-associated virus, vaccinia virus, vesicular stomatitis virus, measles virus, mumps virus, poliovirus, orthomyxovirus, parvovirus, Marabavirus, coxsackievirus, herpesvirus, and lentivirus.

18. An engineered cell expressing the immunosuppressive molecule according to any one of claims 1-14, the nucleic acid molecule according to claim 15, or the vector according to any one of claims 16-17.

19. The engineered cell of claim 18, further **characterized in that** the engineered cell further expresses a functional exogenous receptor.

20. The engineered cell of claim 19, **characterized in that** the functional exogenous receptor is selected from the group consisting of a chimeric antigen receptor, a chimeric T cell receptor, a T cell antigen coupler, and a T cell fusion protein, preferably a chimeric antigen receptor.

21. The engineered cell of claim 19, **characterized in that** the functional exogenous receptor comprises an extracellular domain that specifically recognizes an antigen.

22. The engineered cell of claim 21, **characterized in that** the extracellular domain comprises an antibody that specifically recognizes the antigen or a ligand of the antigen.

23. The engineered cell of claim 22, **characterized in that** the antigen is selected from the group consisting of: ALK, ADRB3, AKAP-4, APRIL, ASGPR1, BCMA, B7H3, B7H4, B7H6, bcr-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CLEC12A, CDH16, CSPG4, CS1, CLL-1, Claudin 6, Claudin18.1, Claudin 18.2, CEA, CEACAM6, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gp130, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region or any combination thereof.

24. The engineered cell of claim 23, **characterized in that** the functional exogenous receptor is a chimeric antigen receptor, and the chimeric antigen receptor targets CD19, CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin18.2, ROR1, EGFRvIII, CS1, BCMA or GPRC5D.

25. The engineered cell of any one of claims 18-24, **characterized in that** the expression of at least one of the endogenous HLA class I gene of the engineered cell is suppressed or silenced.

26. The engineered cell of claim 25, **characterized in that** the endogenous HLA class I gene is selected from the group consisting of HLA-A, HLA-B, HLA-C, and B2M.

27. The engineered cell of any one of claims 18-26, **characterized in that** the expression of at least one of the endogenous HLA class II gene of the engineered cell is suppressed or silenced.

28. The engineered cell of claim 27, **characterized in that** the endogenous HLA class II gene is selected from the group consisting of HLA-DPA, HLA-DQ, HLA-DRA, TAP1, TAP2, LMP2, LMP7, RFX5, RFXAP, RFXANK, and CIITA.

29. The engineered cell of any one of claims 18-28, **characterized in that** the expression of at least one of the endogenous TCR/CD3 gene of the engineered cell is suppressed or silenced.

30. The engineered cell of claim 29, **characterized in that** the TCR/CD3 gene is selected from the group consisting of TRAC, TRBC, CD3γ, CD3δ, CD3ε, CD3ζ, and combinations thereof.

31. The engineered cell of any one of claims 18-30, further **characterized in that**, **characterized in that** the expression of one or more endogenous genes is suppressed or silenced, the endogenous gene is selected from the group consisting of: CD52, GR, dCK, PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

32. The engineered cell of any one of claims 18-31, **characterized in that** the engineered cell is a B cell, a T cell, a macrophage, a dendritic cell, a monocyte, an NK cell, or an NKT cell.

33. The engineered immune cell of claim 32, **characterized in that** the engineered cell is a CD4+CD8+ T cell, a CD4+ T cell, a CD8+ T cell, a CD4-CD8-T cell, a tumor infiltrating cell, a memory T cell, a naive T cell, a γδ-T cell or an αβ-T cell.

34. The engineered cell of any one of claims 18-33, **characterized in that** the engineered cell is derived from cord blood stem cells, progenitor cells, bone marrow stem cells, hematopoietic stem cells, adult stem cells, embryonic stem cells, pluripotent stem cells, or iPSCs.

35. A method for reducing immune rejection of exogenous cells by cells of a subject, **characterized by** expressing the immunosuppressive molecule of any one of claims 1-14, the nucleic acid molecule of claim 15 or the vector of any one of claims 16-17 in the exogenous cells.

36. A method for reducing immune rejection of exogenous cells by cells of a subject, **characterized by** administering the immunosuppressive molecule of any one of claims 1-14 or the engineered cell of any one of claims 18-34 to the subject.

37. A pharmaceutical composition comprising the immunosuppressive molecule of any one of claims 1-14, the nucleic acid molecule of claim 15, the vector of any one of claims 16-17 or the engineered immune cell of any one of claims 18-34, and one or more pharmaceutically acceptable excipients.

38. The pharmaceutical composition of claim 37, **characterized in that** the pharmaceutical composition is used for treating cancer, infection or autoimmune disease.
